# FASCICULE DE BREVET EUROPEEN

(11) **EP 4 202 579 B1**
(45) Date de publication et mention de la délivrance du brevet: **12.08.2026**
(21) Numéro de dépôt: 21216716.7
(22) Date de dépôt: 22.12.2021
(51) Int. Cl.: G04G 21/06, G04G 9/00, G04G 21/04

(54) **PIECE D'HORLOGERIE MUNIE D'UN AFFICHAGE D'UNE SELECTION ADAPTATIVE DE COMMANDES MANUELLES**
UHR MIT EINER ANZEIGE FÜR EINE ADAPTIVE AUSWAHL VON MANUELLEN BEFEHLEN
TIMEPIECE FITTED WITH A DISPLAY OF AN ADAPTIVE SELECTION OF MANUAL CONTROLS

(43) Date de publication de la demande: 28.06.2023
(73) Titulaire: ETA SA Manufacture Horlogère Suisse, 2540 Grenchen (CH)
(72) Inventeur: LAGORGETTE, Pascal, 2502 Bienne (CH)
(74) Mandataire: ICB SA

(56) Documents cités:
- WO-A1-2012/128824
- US-A1- 2015 061 842
- US-A1- 2016 034 887

## Description

### Domaine technique de l'invention

L'invention concerne les pièces d'horlogerie destinées à assister leur utilisateur dans le pilotage d'un autre dispositif.

Du fait de leur disponibilité immédiate et de leur port souvent permanent pour l'utilisateur, les pièces d'horlogerie sont idéalement placées pour servir de télécommande. De nouveaux types de pièces d'horlogerie constituent ainsi un outil de prédilection pour recueillir des commandes de l'utilisateur à destination d'autres dispositifs. De tels dispositifs peuvent par exemple inclure un téléviseur, un système audio, une installation domotique ou des écouteurs portatifs.

De nouvelles pièces d'horlogerie sont apparues et proposent un microphone destiné à recueillir des commandes vocales et une interface radio pour transmettre la commande vocale au dispositif concerné ou à une passerelle. Un dispositif de traitement, soit dans la pièce d'horlogerie, soit déporté, convertit le signal sonore reçu sur le microphone en une commande numérique pour le dispositif concerné. La disponibilité d'une commande vocale s'avère particulièrement utile lors de la conduite d'une automobile, lors d'une activité sportive, lors d'un déplacement à vélo ou même lors d'une activité domestique qui peut immobiliser les mains de l'utilisateur.

Dans un certain nombre de situations, la commande vocale peut s'avérer inadaptée. Ainsi, lorsque l'environnement s'avère trop bruyant, le signal sonore reçu sur le microphone peut présenter une qualité insuffisante pour que la commande vocale puisse être convertie en commande numérique pour le dispositif concerné. A contrario, l'environnement peut imposer le silence, par exemple dans un lieu public ou dans les transports en commun.

On connait dans l'état de la technique des pièces d'horlogerie permettant à l'utilisateur de pouvoir saisir des commandes. De telles pièces d'horlogerie disposent classiquement d'un écran d'affichage numérique et d'une interface de sélection, par exemple sous forme d'une détection tactile à la surface de l'écran d'affichage numérique. Un tel écran est susceptible d'afficher une interface de commande comprenant plusieurs propositions de commandes à destination de dispositifs pilotés. Lorsqu'une des commandes affichées est sélectionnée par l'intermédiaire de l'interface de sélection, elle est ensuite émise à destination du dispositif piloté.

Cependant, une telle pièce d'horlogerie présente de nombreux inconvénients. En effet, avec peu de surface disponible sur l'écran d'affichage numérique, l'affichage des propositions de commandes peut s'avérer assez peu adapté, de fait peu de commandes peuvent être proposées en simultané sur cet écran. De plus, si un certain nombre de ces commandes sont indisponibles ou inappropriées, le mode de sélection manuel de commande s'avère fastidieux à utiliser.

On connait également dans l'état de la technique le document de brevet n°WO 2012/128824A1 qui décrit un exemple de montre permettant d'assister son porteur dans le pilotage d'un autre dispositif qui présente des inconvénients similaires à ceux évoqués précédemment. Le document US 2016/034887 A1 divulgue une montre avec interface graphique structurée en listes de commandes avec une adaptation de cette interface en fonction de l'historique et du contexte.

L'invention vise à résoudre un ou plusieurs de ces inconvénients.

### Résumé de l'invention

Un des buts de l'invention est de disposer d'une pièce d'horlogerie qui est apte à afficher une interface de commande d'un dispositif externe ou piloté qui est claire, précise, intuitive, efficace et/ou adaptée au plus près aux besoins d'assistance de l'utilisateur de cette pièce dans le cadre du contrôle de ce dispositif externe.

Un autre des buts de l'invention est de disposer d'une pièce d'horlogerie qui est apte à générer de manière dynamique cette interface de commande en fonction des besoins d'assistance de l'utilisateur de cette pièce pour le contrôle de ce dispositif externe.

Dans ce dessein, l'invention concerne une pièce d'horlogerie destinée à émettre des commandes à destination d'un dispositif externe, comprenant :
- un afficheur numérique ;
- un circuit de communication sans fil avec un dispositif externe ;
- une interface de sélection manuelle ;
- un microphone ;
- une unité de traitement configurée pour :
   ∘ émettre une commande à destination d'un dispositif externe en fonction d'un signal sonore reçu sur le microphone, par l'intermédiaire du circuit de communication sans fil ;
   ∘ en fonction d'un historique de commandes sélectionnées antérieurement, de critères environnementaux, de critères comportementaux, de critères physiologiques du porteur de la pièce d'horlogerie, ou de critères d'état du dispositif externe, définir une liste de propositions de commandes à afficher ;
   ∘ afficher sur l'afficheur numérique une interface de commande comprenant lesdites propositions de commandes de la liste définie ;
   ∘ identifier la sélection d'une commande affichée sur l'afficheur numérique, par l'intermédiaire de l'interface de sélection manuelle ;
   ∘ émettre une commande à destination d'un dispositif externe en fonction de la commande dont la sélection est identifiée, par l'intermédiaire du circuit de communication sans fil ;
   ∘ mettre en œuvre un algorithme d'apprentissage qui est apte à participer à la génération de manière dynamique sur l'afficheur numérique de ladite interface de commande comprenant les propositions de commandes, l'unité de traitement exécutant cet algorithme est apte à générer et à émettre de manière proactive au moins une commande à destination d'au moins un dispositif externe évitant ainsi toute interaction de l'utilisateur avec l'interface de sélection.

Selon une variante, la pièce d'horlogerie comprend en outre une interface de mesure de données physiologiques d'un porteur de la pièce d'horlogerie, ladite unité de traitement étant configurée pour définir la liste de propositions de commandes à afficher en fonction de critères physiologiques du porteur de la pièce d'horlogerie.

Selon une autre variante, la pièce d'horlogerie comprend en outre un dispositif de géolocalisation, ladite unité de traitement étant configurée pour définir la liste de propositions de commandes à afficher en fonction de la position de la pièce d'horlogerie déterminée par le dispositif de géolocalisation.

Selon une autre variante, la pièce d'horlogerie comprend en outre une mémoire mémorisant une ou plusieurs des dernières commandes émises, ladite unité de traitement étant configurée pour définir la liste de propositions de commandes à afficher en incluant une ou plusieurs des dernières commandes mémorisées ou une ou plusieurs commandes contraires à ces dernières commandes.

Selon une autre variante, la pièce d'horlogerie comprend en outre un accéléromètre, ladite unité de traitement étant configurée pour définir la liste de propositions de commandes à afficher en fonction des accélérations mesurées par l'accéléromètre.

Selon encore une variante, ladite unité de traitement est configurée pour définir la liste de propositions de commandes à afficher en fonction d'un critère d'état détecté pour un dispositif externe positionné à proximité de la pièce d'horlogerie.

Selon encore une autre variante, l'interface de sélection manuelle est une dalle tactile associée à l'afficheur numérique.

### Brève description des figures

D'autres particularités et avantages ressortiront clairement de la description qui en est faite ci-après, à titre indicatif et nullement limitatif, en référence aux figures annexées, dans lesquelles :
- la figure 1 est une vue de dessus d'un exemple de montre pour laquelle l'invention peut être mise en œuvre ;
- la figure 2 est une représentation d'un exemple de communication directe d'une montre avec plusieurs dispositifs externes ;
- la figure 3 est une représentation d'un exemple de communication d'une montre avec plusieurs dispositifs externes par l'intermédiaire d'un smartphone ;
- la figure 4 est une vue de dessus grossie de différentes zones d'affichage au niveau de l'afficheur de la montre de la figure 1 ;
- la figure 5 est une vue de dessus grossie d'un premier niveau d'affichage de commandes ;
- la figure 6 est une vue de dessus grossie d'un deuxième niveau d'affichage de commandes ;
- la figure 7 est illustration schématique d'un exemple d'arborescence de l'affichage des commandes ;
- la figure 8 illustre la montre de la figure 1 en communication avec un serveur.

### Description détaillée de l'invention

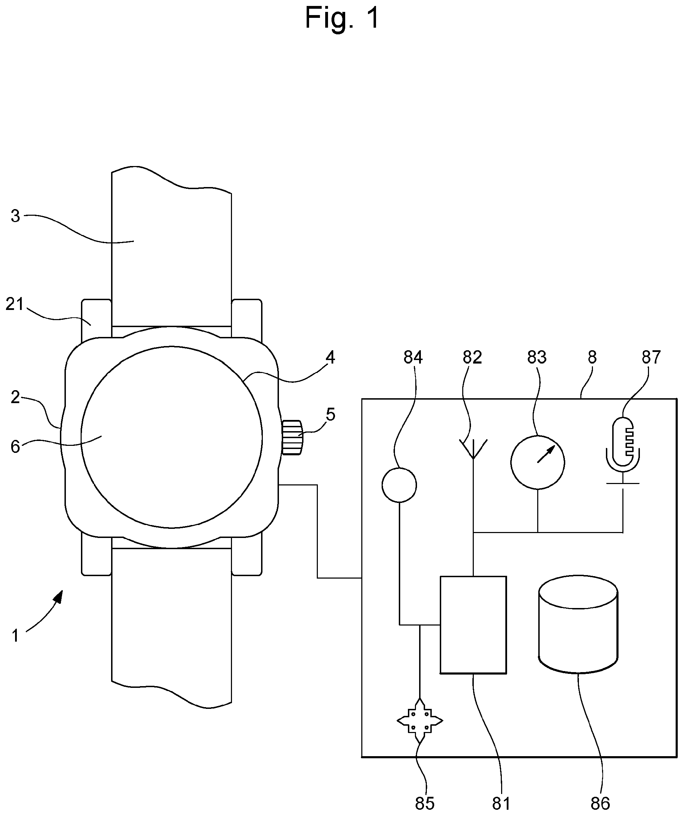

La figure 1 illustre une représentation schématique d'une pièce d'horlogerie sous la forme d'une montre 1 en vue de dessus, fournie dans un but illustratif et pour laquelle l'invention est susceptible d'être mise en œuvre. La montre 1 comporte un boîtier 2 muni d'une carrure. La montre 1 comporte ici un bracelet 3 fixé au boîtier 2. A cet effet, la montre 1 comporte de part et d'autre de sa carrure, deux cornes 21 agencées chacune pour fixer un brin distinct du bracelet 3. La montre 1 comporte ici un élément activable 5 positionné sur le côté du boîtier 2, cet élément 5 peut être par exemple un bouton poussoir, une couronne ou encore une couronne à poussoir.

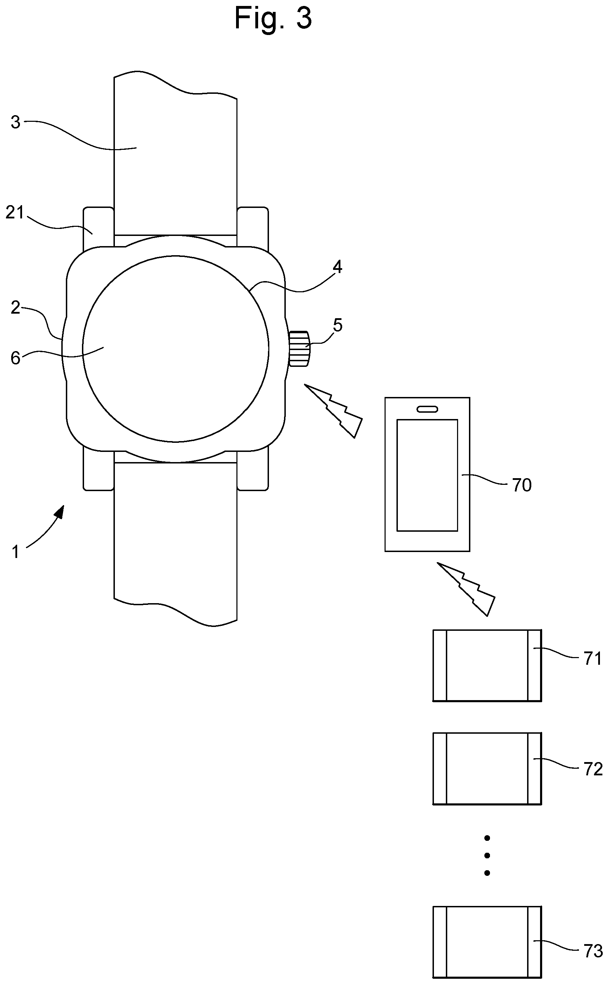

Sur la figure 3, le boîtier 2 de la montre 1 comporte un volume d'accueil 4 défini par la carrure et logeant des moyens numériques. Le volume d'accueil 4 de la montre 1 est fermé par un afficheur numérique 6. L'afficheur 6 peut être supporté de façon connue en soit par la carrure et fixée de manière étanche à celle-ci par un procédé adapté.

Outre des fonctions de base d'indication chronologique, l'afficheur 6 peut être utilisé par des moyens numériques 8 pour afficher une interface de commande comprenant des propositions de commande à destination de dispositifs externes ou pilotés. Les moyens numériques 8 peuvent être intégrés sur un même circuit électronique ou être décomposés en différents circuits ou composants électroniques. Les moyens numériques 8 comportent ici une unité de traitement 81 ou contrôleur, un circuit de communication sans fil 82, un dispositif de géolocalisation 83, une interface de mesure ou de réception de données physiologiques 84, et un accéléromètre 85. Les moyens numériques 8 peuvent également comprendre une base de données 86 (par exemple stockée dans une mémoire non volatile) et un microphone 87. Dans un souci d'exhaustivité, les moyens numériques 8 incluent ici une combinaison d'une multitude de fonctions, l'invention pouvant bien entendu être mise en œuvre avec seulement une partie d'entre elles.

Ainsi que nous l'avons déjà évoqué, le dispositif externe est un dispositif électronique qui peut être piloté/contrôlé à distance au travers d'une liaison sans fil par la présente montre. Il peut s'agir de manière non limitative et non exhaustive d'un téléviseur, d'un système audio et/ou vidéo, d'une installation domotique, d'un jouet ou encore d'écouteurs portatifs, etc...

L'unité de traitement 81 est notamment configurée pour émettre une commande à destination d'au moins un dispositif externe en fonction d'un signal sonore reçu sur le microphone 87. Une telle commande est émise par l'intermédiaire du circuit de communication sans fil 82. L'unité de traitement 81 peut notamment être pourvue d'une fonction de reconnaissance vocale pour identifier des phonèmes prononcés par un utilisateur et identifier une commande potentielle dans une liste de commandes possibles pour un dispositif externe. La commande ainsi identifiée est émise par l'intermédiaire du circuit de communication sans fil 82.

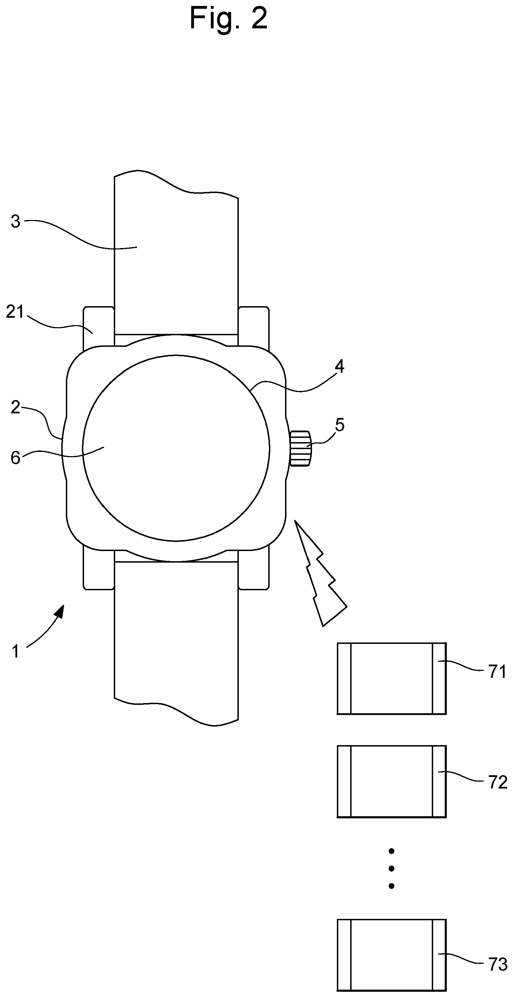

La figure 2 illustre schématiquement la possibilité pour la montre 1 d'émettre des commandes de pilotage de différents dispositifs 71, 72 ou 73, par l'intermédiaire de son circuit de communication sans fil 82. La montre 1 est ici autonome et apte à envoyer de telles commandes directement aux dispositifs 71 à 73.

La figure 3 illustre schématiquement la possibilité pour la montre 1 d'émettre des commandes de pilotage des différents dispositifs 71 à 73, en utilisant un smartphone ou une tablette 70 comme relais, par l'intermédiaire de son circuit de communication sans fil. Le smartphone ou la tablette 70 sont configurés pour exécuter différentes applications, chacune de ces applications étant configurée pour commander un dispositif 71 à 73 respectif. Les applications du smartphone ou de la tablette 70 sont alors utilisées comme relais des commandes émises par la montre à destination des dispositifs 71 à 73.

En présence d'un environnement bruyant ou nécessitant le silence, l'utilisateur dispose également d'une interface de sélection manuelle de commandes à destination d'un dispositif externe.

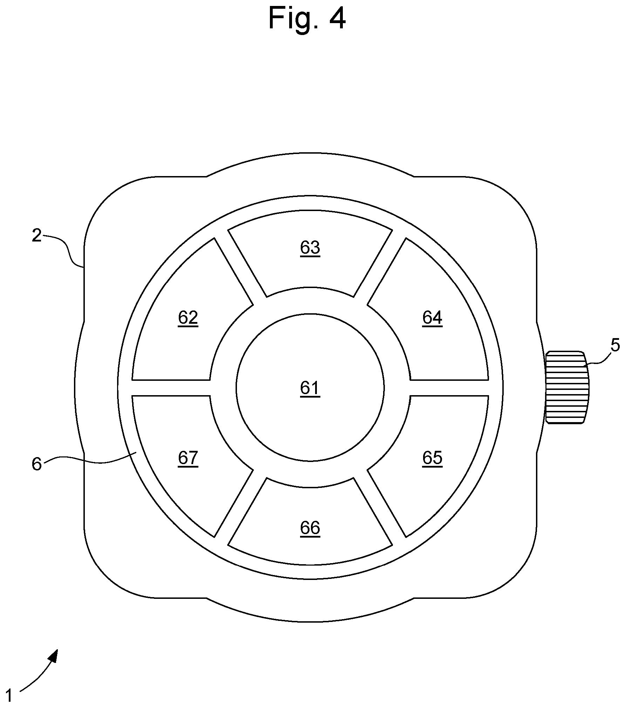

La figure 4 est une vue de dessus de la montre 1 au niveau de son afficheur numérique 6. L'afficheur numérique 6 comporte ici plusieurs zones d'affichage 61 à 67 qui peuvent être utilisées par l'unité de traitement 81 pour afficher des propositions de commande. L'unité de traitement 81 pourra être configurée pour afficher des zones d'affichage différentes de cette illustration en nombre et en répartition, en fonction notamment du nombre de commandes proposées. On notera que ces zones d'affichages sont visibles au travers de différentes portions activables/sélectionnables correspondantes de l'interface de sélection manuelle.

Cette interface de sélection manuelle d'une commande peut par exemple être formée d'une surface tactile de l'afficheur 6, permettant de sélectionner une commande par une pression directement sur une de ses portions activables/sélectionnables correspondant à une des zones d'affichage 61 à 67. L'interface de sélection manuelle peut également inclure l'élément activable 5 pour présélectionner séquentiellement les différentes zones d'affichage (par exemple par des appuis brefs), puis pour sélectionner une commande correspondant la zone d'affichage présélectionnée (par exemple par un appui prolongé).

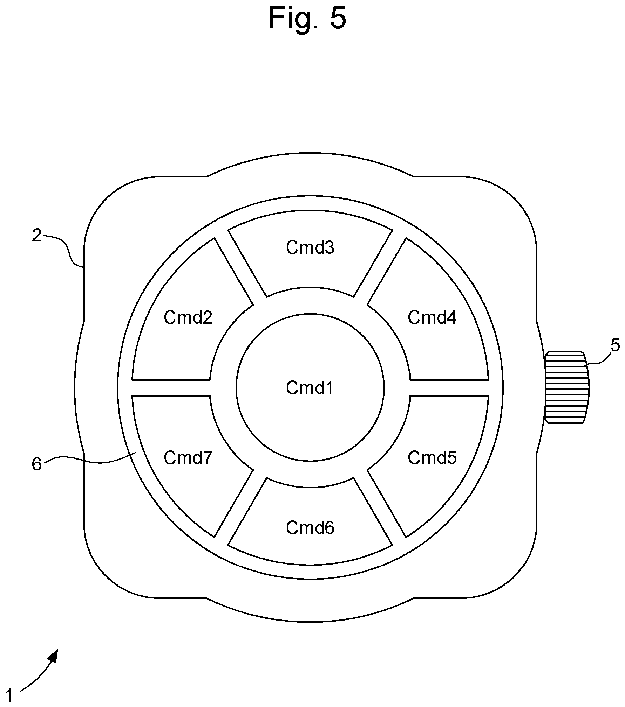

Comme illustré à la figure 5, l'afficheur 6 affiche des commandes Cmd1, Cmd2, Cmd3, Cmd4, Cmd5, Cmd6 et Cmd7 dans les différentes zones d'affichage. Ces commandes peuvent être un premier niveau de commande proposées, par exemple la sélection d'un dispositif externe ou un raccourci vers la dernière commande sélectionnée ou l'inverse de la dernière commande sélectionnée. L'affichage des dernières commandes sélectionnées peut se faire soit en mentionnant un tel libellé (« dernière commande », « avant-dernière commande ») soit en rappelant explicitement la teneur de ces commandes.

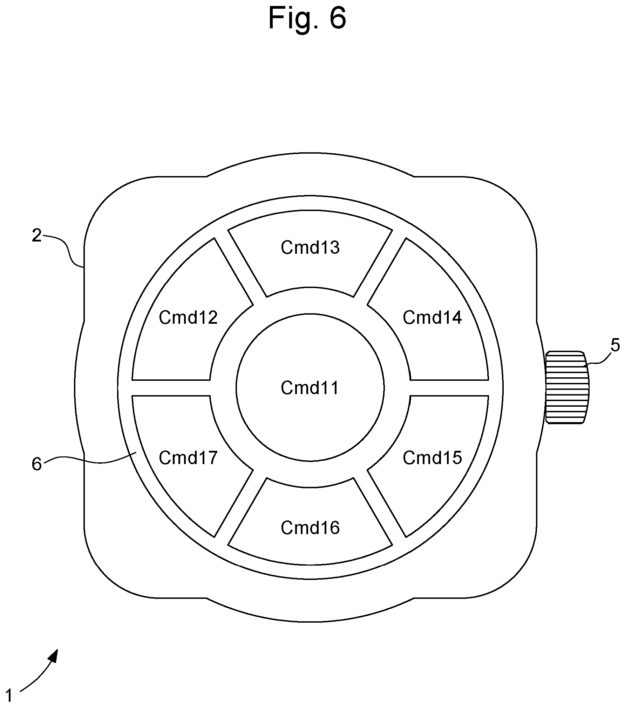

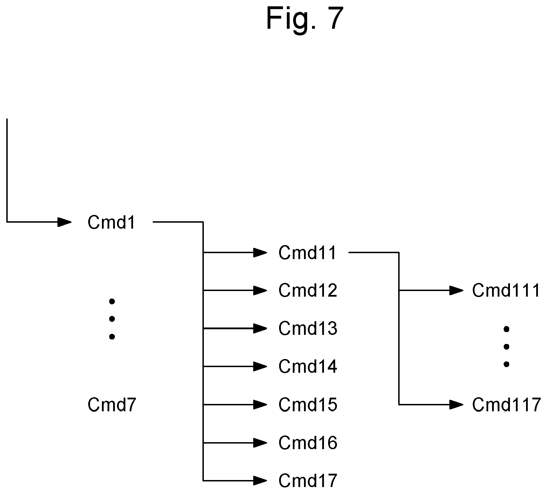

En référence à la figure 7, par une sélection d'une des commandes, l'afficheur 6 peut être amené à afficher d'autres commandes selon une arborescence. Ainsi, par la sélection d'une des commandes Cmd1 (par exemple un identifiant de dispositif externe), l'afficheur 6 peut afficher d'autres commandes Cmd11, Cmd12, Cmd13, Cmd14, Cmd15, Cmd16 et Cmd17, comme illustré à la figure 6 (pour proposer par exemple des commandes plus précisément destinées au dispositif externe sélectionné). On comprend que certaines ou toutes les commandes Cmd11, Cmd12, Cmd13, Cmd14, Cmd15, Cmd16 et Cmd17 sélectionnées peuvent aussi comprendre d'autres commandes comme par exemple la commande Cmd11 qui comporte les commandes Cmd111 à Cmd117.

La succession d'affichages peut correspondre à une arborescence telle qu'illustrée à titre d'exemple à la figure 7. Le premier niveau de l'arborescence peut proposer des commandes spécifiques, par exemple choisies dans la liste suivante : lancer, stopper, allumer, éteindre, baisser. Le second niveau de l'arborescence peut proposer des commandes associées à un paramètre, par exemple choisi dans la liste suivante : volume, luminosité, vitesse. Le troisième niveau de l'arborescence peut proposer différents dispositifs externes disponibles pour l'application de la commande, par exemple choisi dans la liste suivante : un téléviseur, une enceinte connectée, des écouteurs, etc. Le quatrième niveau de l'arborescence peut proposer différentes amplitudes de certaines commandes, par exemple 30%, moitié, 80%, correspondant à des commande d'augmentation ou de baisse par exemple.

Avec un tel système d'arborescence, on peut prévoir que la commande soit émise à destination d'un dispositif externe après la sélection d'une combinaison de plusieurs commandes successives par l'intermédiaire de l'interface de sélection manuelle.

L'unité de traitement 81 est en outre configurée pour tenir compte d'un des critères suivants pour définir une liste de propositions de commandes à afficher (par exemple pour l'ensemble des commandes Cmd1, Cmd2, Cmd3, Cmd4, Cmd5, Cmd6 et Cmd7) : un historique de commandes sélectionnées antérieurement par l'utilisateur, des critères environnementaux, des critères physiologiques du porteur de la montre 1, des critères comportementaux du porteur de la montre 1 ou des critères d'état du dispositif externe. L'unité de traitement 81 est en outre configurée pour afficher l'interface de commande sur l'afficheur numérique 6 comprenant les propositions de commandes définies dans cette liste. L'unité de traitement 81 est en outre configurée pour ensuite identifier la sélection d'une des commandes affichées sur l'afficheur 6, par l'intermédiaire de son interface de sélection manuelle. L'unité de traitement 81 est alors configurée pour émettre une commande à destination d'un des dispositifs externes 71 à 73 ou le smartphone 70 par exemple, en fonction de la commande dont on a identifié la sélection. Cette émission de commande est effectuée par l'intermédiaire du circuit de communication sans fil 82.

Ainsi, la montre 1 et en particulier son unité de traitement 81 qui est connectée à l'interface de sélection, dispose d'une possibilité de récupérer des commandes manuelles lorsque la réception d'une commande sonore par le microphone n'est pas disponible ou souhaitable. En outre, la récupération d'une commande manuelle dispose d'une ergonomie optimisée pour ne pas rendre la manipulation fastidieuse, du fait d'une gestion intelligente d'au moins une des commandes affichées sur l'afficheur 6.

Dans ce contexte, l'unité de traitement 81 de la montre est apte à mettre en œuvre un programme informatique tel qu'un algorithme d'apprentissage qui est apte à générer de manière dynamique sur l'afficheur numérique 6 l'interface de commande comprenant les propositions de commandes à destination de l'utilisateur. De manière alternative ou complémentaire, l'unité de traitement 81 exécutant cet algorithme est apte à générer et à émettre de manière proactive au moins une commande à destination d'au moins un dispositif externe évitant ainsi toute interaction de l'utilisateur avec l'interface de sélection. On notera qu'une telle montre peut piloter/contrôler un ou plusieurs dispositifs externes notamment simultanément.

Un tel algorithme d'apprentissage est connu sous l'expression anglaise « machine learning algorithm ». Plus particulièrement, il s'agit ici d'un algorithme d'apprentissage relatif à la sélection passée des commandes par l'interface de sélection manuelle. Cet algorithme peut faire l'objet d'un entraînement automatique encore appelé apprentissage automatique qui est de préférence supervisé. A partir de données récupérées dans la base de données 86, l'unité de traitement 81 peut être entrainée sur la base d'au moins un réseau de neurones et/ou d'une fonction analytique et/ou d'un principe de régression polynomial. Dans le cadre de son exécution, l'algorithme est apte générer des propositions de commande à afficher et aussi apte à apprendre si certaines commandes ont une plus grande possibilité d'être sélectionnées dans un contexte donné, en combinant par exemple les commandes sélectionnées par le passé avec d'autres données telles que des critères environnementaux, des critères physiologiques du porteur de la montre 1, des critères comportementaux du porteur de la montre 1 ou des critères d'état d'un dispositif externe.

Dans le présent mode de réalisation, cette unité de traitement 81 en exécutant cet algorithme est apte à générer l'historique de commandes sélectionnées antérieurement par l'utilisateur. Pour ce faire l'unité de traitement 81 est apte à détecter à partir de l'interface de sélection, au moins une portion activable correspondant à au moins une zone d'affichage 61 à 67 qui est attribuée à la dernière commande sélectionnée par l'utilisateur, et qui peut avoir la plus grande probabilité de sélection ou à l'inverse permettre de déterminé qu'une commande contraire à cette dernière commande pourrait avoir cette plus grande probabilité de sélection. Pour ce faire, l'algorithme d'apprentissage peut prendre en compte des commandes sélectionnées antérieurement ainsi que des éléments de contexte. A cet effet, la base de données 86 pourra mémoriser un historique d'un certain nombre de dernières commandes sélectionnées. Le contenu de la base de données 86 pourra par exemple être utilisé comme contenu d'apprentissage pour un tel algorithme d'apprentissage.

Dans ce contexte, des critères physiologiques du porteur de la montre 1 peuvent être utilisées par cet algorithme mis en œuvre par l'unité de traitement 81. En effet, l'unité de traitement 81 peut être configurée pour utiliser des données fournies par l'interface de mesure de données physiologiques 84. Entre autres paramètres pouvant être fournis pour tenir compte de ces données physiologiques, on peut par exemple envisager le pouls, la saturation du sang en oxygène, l'impédance de la peau, la tension artérielle, le rythme respiratoire, l'arythmie respiratoire, la température cutanée, le taux de sudation, ou le débit sanguin, etc. On peut par exemple tenir compte d'un ralentissement du rythme cardiaque en dessous d'un certain seuil pour proposer une commande d'extinction d'un téléviseur, pour anticiper un assoupissement de l'utilisateur. Une interface de mesure ou de réception de données physiologiques est connue en soi de l'homme du métier.

L'algorithme exécuté par l'unité de traitement 81 peut également prendre en compte des critères environnementaux. Plus précisément, l'unité de traitement 81 peut être configurée pour utiliser des données fournies par les moyens numériques 8. A titre d'exemple, ces critères de environnementaux peuvent comprendre un niveau de bruit fourni par un microphone 87 connecté à l'unité de traitement 81, un niveau de lumière ambiante fourni par un capteur photosensible (non représenté), une position de la montre 1 calculée par l'accéléromètre 85, ou des conditions atmosphériques déterminées par un capteur de pression (non représenté) ou par le dispositif de géolocalisation 83.

Dans ce mode de réalisation l'unité de traitement 81 exécutant cet algorithme peux aussi prendre en compte des critères d'état du dispositif externe, comme par exemple l'état d'alimentation d'un tel dispositif - marche, veille, arrêt -, la distance entre la montre 1 et ce dispositif externe, des paramètres de fonctionnement (s'il s'agit d'un téléviseur : niveau sonore du téléviseur par rapport au bruit ambiant, niveau de luminosité du téléviseur par rapport à la luminosité ambiante, durée de marche, etc...). Une commande d'allumage peut par exemple être proposée pour une application domotique, si les éclairages environnants sont identifiés comme éteints et que la montre est déterminée comme présente à une distance réduite de ces éclairages.

L'unité de traitement 81 exécutant cet algorithme peut aussi prendre en compte des critères comportementaux, tels que des mesures du niveau d'activité de l'utilisateur au moyen de l'accéléromètre 85 ou du dispositif de géolocalisation 83 ou encore l'identification d'un mouvement ou d'une série de mouvements réalisée par l'utilisateur/porteur de cette montre à partir de l'accéléromètre 85 par exemple pour détecter une pratique sportive ou un déplacement. En détectant une pratique sportive, la liste de commandes proposées peut être établie en estimant pour un port d'écouteurs par l'utilisateur le moment où il souhaite sélectionner une commande.

Avantageusement, on peut envisager que l'unité de traitement 81 soit configurée pour envoyer de façon proactive une des commandes proposées à destination d'un dispositif externe. Par exemple, lors de la détection du ralentissement du rythme cardiaque, après avoir proposé une commande d'extinction d'un téléviseur, l'unité de traitement 81 pourra envoyer cette commande d'extinction de façon autonome après une durée d'affichage sur l'afficheur numérique 6.

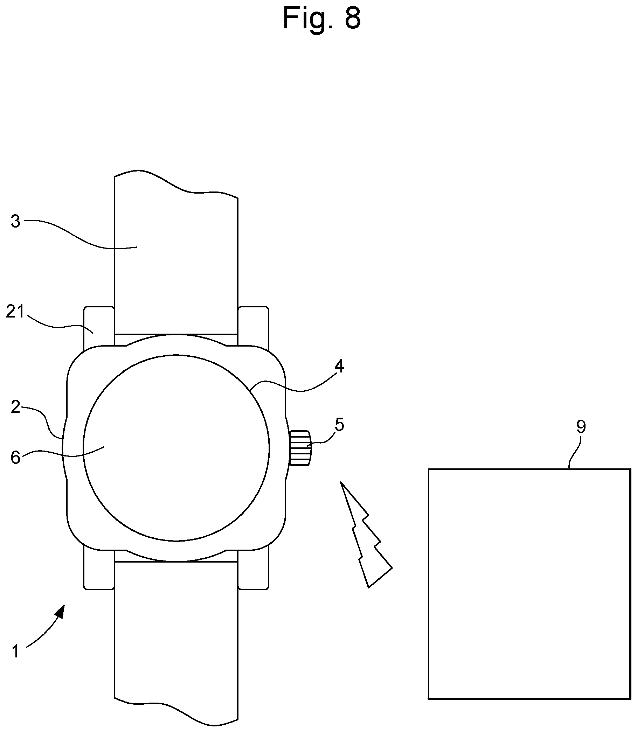

L'algorithme d'apprentissage peut être mis en œuvre de façon déportée, par exemple sur le smartphone 70 ou sur un serveur 9 en communication avec la montre 1 (comme illustré à la figure 8), afin de disposer potentiellement de ressources supérieures. Les données d'entrainement pouvant être basées sur des données disponibles en local, l'apprentissage peut être optimal et dédié à l'utilisateur de montre 1.

L'afficheur 6 peut être un afficheur général, utilisé séquentiellement par différentes fonctions de la montre 1. On peut également envisager que l'afficheur 6 soit un afficheur général utilisé simultanément par différentes fonctions de la montre.

L'unité de traitement 81 peut être mise en œuvre sous la forme d'un microphone contrôleur ou d'un processeur numérique. La fonction de l'unité de traitement 81 peut partiellement être déportée hors de la montre 1. Par exemple, des fonctions de reconnaissance vocale ou des algorithmes d'apprentissage définissant le modèle prédictif de sélection des commandes peuvent être mises en œuvre sur un serveur distant, ou sur le smartphone 70 pouvant disposer de davantage de ressources. Pour la reconnaissance vocale, le signal sonore reçu par la montre 1 peut être transmis à un dispositif déporté par l'intermédiaire du circuit de communication sans fil 82, puis ce dispositif déporté peut renvoyer à la montre 1 la transcription du signal sonore en une suite de caractères ou de mots identifiés par une fonction de reconnaissance vocale.

Le circuit de communication sans fil 82 est configuré pour communiquer sans fil avec un dispositif externe tel que cité précédemment. Le circuit de communication 82 pourra communiquer selon tout protocole radio approprié, de façon connue en soi.

Le dispositif de géolocalisation 83 est un circuit connu en soi permettant de générer des informations de localisation de la montre 1. Le dispositif de géolocalisation 83 peut par exemple déterminer la localisation instantanée de la montre 1 par communication avec un réseau satellitaire tel que le Global Positioning Service ou Galileo.

### Nomenclature

- 1.: montre
- 2.: boîtier
- 3.: bracelet
- 4.: volume d'accueil
- 5.: élément activable
- 6: afficheur numérique
- 7.: afficheur d'exposition
- 8: moyens numériques
- 9: serveur
- 21.: cornes
- 61.: zone d'affichage
- 62.: zone d'affichage
- 63.: zone d'affichage
- 64.: zone d'affichage
- 65.: zone d'affichage
- 66.: zone d'affichage
- 67: zone d'affichage
- 70: smartphone
- 71: dispositif externe
- 72: dispositif externe
- 73: dispositif externe
- 81.: unité de traitement
- 82: circuit de communication sans fil
- 83: dispositif de géolocalisation
- 84: interface de données physiologiques
- 85: accéléromètre
- 86: base de données
- 87: microphone
- 91: interface de communication
- 92: base de données

## Revendications

1. Pièce d'horlogerie (1) destinée à émettre des commandes à destination d'un dispositif externe (70, 71, 72, 73), **caractérisée en ce qu'**elle comprend :
- un afficheur numérique (6) ;
- un circuit de communication sans fil (82) avec un dispositif externe ;
- une interface de sélection manuelle (5, 6) ;
- un microphone (87) ;
- une unité de traitement (81) configurée pour :
∘ émettre une commande à destination d'un dispositif externe (70, 71, 72, 73) en fonction d'un signal sonore reçu sur le microphone (87), par l'intermédiaire du circuit de communication sans fil ;
∘ en fonction d'un historique de commandes sélectionnées antérieurement, de critères environnementaux, de critères comportementaux, de critères physiologiques du porteur de la pièce d'horlogerie, ou de critères d'état du dispositif externe, définir une liste de propositions de commandes à afficher ;
∘ afficher sur l'afficheur numérique (6) une interface de commande comprenant lesdites propositions de commandes (cmd1, cmd2, cmd3, cmd4, cmd5, cmd6, cmd7) de la liste définie ;
∘ identifier la sélection d'une commande affichée sur l'afficheur numérique, par l'intermédiaire de l'interface de sélection manuelle ;
∘ émettre une commande à destination d'un dispositif externe (70, 71, 72, 73) en fonction de la commande dont la sélection est identifiée, par l'intermédiaire du circuit de communication sans fil (82),
**caractérisé en ce que** l'unité de traitement (81) est configurée pour mettre en œuvre un algorithme d'apprentissage qui est apte à participer à la génération de manière dynamique sur l'afficheur numérique (6) de ladite interface de commande comprenant les propositions de commandes, l'unité de traitement (81) exécutant cet algorithme est apte à générer et à émettre de manière proactive au moins une commande à destination d'au moins un dispositif externe évitant ainsi toute interaction de l'utilisateur avec l'interface de sélection.

2. Pièce d'horlogerie (1) selon la revendication 1, **caractérisée en ce qu'**elle comprend une interface de mesure de données physiologiques d'un porteur de la pièce d'horlogerie (84), ladite unité de traitement (81) étant configurée pour définir la liste de propositions de commandes à afficher en fonction de critères physiologiques du porteur de la pièce d'horlogerie.

3. Pièce d'horlogerie (1) selon la revendication 1 ou 2, **caractérisée en ce qu'**elle comprend un dispositif de géolocalisation (83), ladite unité de traitement (81) étant configurée pour définir la liste de propositions de commandes à afficher en fonction de la position de la pièce d'horlogerie déterminée par le dispositif de géolocalisation (83).

4. Pièce d'horlogerie (1) selon l'une quelconque des revendications précédentes, **caractérisée en ce qu'**elle comprend une mémoire mémorisant une ou plusieurs des dernières commandes émises, ladite unité de traitement (81) étant configurée pour définir la liste de propositions de commandes à afficher en incluant une ou plusieurs des dernières commandes mémorisées ou une ou plusieurs commandes contraires à ces dernières commandes.

5. Pièce d'horlogerie (1) selon l'une quelconque des revendications précédentes, **caractérisée en ce qu'**elle comprend un accéléromètre (85), ladite unité de traitement (81) étant configurée pour définir la liste de propositions de commandes à afficher en fonction des accélérations mesurées par l'accéléromètre (85).

6. Pièce d'horlogerie (1) selon l'une quelconque des revendications précédentes, **caractérisée en ce que** ladite unité de traitement (81) est configurée pour définir la liste de propositions de commandes à afficher en fonction d'un critère d'état détecté pour un dispositif externe positionné à proximité de la pièce d'horlogerie.

7. Pièce d'horlogerie (1) selon l'une quelconque des revendications précédentes, **caractérisée en ce que** l'interface de sélection manuelle est une dalle tactile associée à l'afficheur numérique (6).

## Patentansprüche

1. Zeitmessgerät (1) zum Senden von Befehlen an eine externe Vorrichtung (70, 71, 72, 73), **dadurch gekennzeichnet, dass** es Folgendes umfasst:
- eine digitale Anzeige (6);
- einen drahtlosen Kommunikationsschaltkreis (82) mit einer externen Vorrichtung;
- eine manuelle Auswahlschnittstelle (5, 6);
- ein Mikrofon (87);
- eine Verarbeitungseinheit (81), die konfiguriert ist, um:
∘ einen Befehl an eine externe Vorrichtung (70, 71, 72, 73) in Abhängigkeit von einem am Mikrofon (87) empfangenen akustischen Signal über den drahtlosen Kommunikationsschaltkreis zu senden;
∘ in Abhängigkeit von einem Verlauf zuvor ausgewählter Befehle, von Umweltkriterien, von Verhaltenskriterien, von physiologischen Kriterien des Trägers des Zeitmessgeräts oder von Zustandskriterien der externen Vorrichtung eine Liste von anzuzeigenden Befehlsvorschlägen zu definieren;
∘ auf der digitalen Anzeige (6) eine Befehlsschnittstelle anzuzeigen, die diese Befehlsvorschläge (cmd1, cmd2, cmd3, cmd4, cmd5, cmd6, cmd7) der definierten Liste umfasst;
∘ die Auswahl eines auf der digitalen Anzeige angezeigten Befehls über die manuelle Auswahlschnittstelle zu identifizieren;
∘ einen Befehl an eine externe Vorrichtung (70, 71, 72, 73) in Abhängigkeit von dem Befehl, dessen Auswahl identifiziert wird, über den drahtlosen Kommunikationsschaltkreis (82) zu senden,
**dadurch gekennzeichnet, dass** die Verarbeitungseinheit (81) dafür konfiguriert ist, einen Lernalgorithmus umzusetzen, der geeignet ist, an der dynamischen Erzeugung auf der digitalen Anzeige (6) der genannten Befehlsschnittstelle mit den Befehlsvorschlägen mitzuwirken, wobei die Verarbeitungseinheit (81), die diesen Algorithmus ausführt, in der Lage ist, proaktiv mindestens einen Befehl an mindestens eine externe Vorrichtung zu erzeugen und zu senden, wodurch jede Interaktion des Benutzers mit der Auswahlschnittstelle vermieden wird.

2. Zeitmessgerät (1) nach Anspruch 1, **dadurch gekennzeichnet, dass** es eine Schnittstelle zur Messung physiologischer Daten eines Trägers der Uhr (84) umfasst, wobei die Verarbeitungseinheit (81) dafür konfiguriert ist, die anzuzeigende Liste von Steuerungsvorschlägen in Abhängigkeit von physiologischen Kriterien des Uhrträgers festzulegen.

3. Zeitmessgerät (1) nach Anspruch 1 oder 2, **dadurch gekennzeichnet, dass** es eine Geolokalisierungsvorrichtung (83) umfasst, wobei die Verarbeitungseinheit (81) konfiguriert ist, die Liste der anzuzeigenden Befehlsvorschläge in Abhängigkeit von der durch die Geolokalisierungsvorrichtung (83) bestimmten Position des Zeitmessgeräts zu definieren.

4. Zeitmessgerät (1) nach einem beliebigen der vorstehenden Ansprüche, **dadurch gekennzeichnet, dass** es einen Speicher umfasst, der einen oder mehrere der zuletzt gesendeten Befehle speichert, wobei die Verarbeitungseinheit (81) dafür konfiguriert ist, die Liste der anzuzeigenden Befehlsvorschläge unter Einbeziehung eines oder mehrerer der zuletzt gespeicherten Befehle oder eines oder mehrerer diesen letzten Befehlen entgegenstehender Befehle zu definieren.

5. Zeitmessgerät (1) nach einem beliebigen der vorstehenden Ansprüche, **dadurch gekennzeichnet, dass** es einen Beschleunigungsmesser (85) umfasst, wobei die Verarbeitungseinheit (81) dafür konfiguriert ist, die Liste der anzuzeigenden Befehlsvorschläge in Abhängigkeit von den durch den Beschleunigungsmesser (85) gemessenen Beschleunigungen zu definieren.

6. Zeitmessgerät (1) nach einem beliebigen der vorstehenden Ansprüche, **dadurch gekennzeichnet, dass** die Verarbeitungseinheit (81) konfiguriert ist, die Liste der anzuzeigenden Befehlsvorschläge in Abhängigkeit von einem für eine externe Vorrichtung, die in der Nähe des Zeitmessgeräts angeordnet ist, ermittelten Zustandskriterium zu definieren.

7. Zeitmessgerät (1) nach einem beliebigen der vorstehenden Ansprüche, **dadurch gekennzeichnet, dass** die manuelle Auswahlschnittstelle eine mit der digitalen Anzeige (6) verbundene Touchscreen-Fläche ist.

## Claims

1. Timepiece (1) designed to send commands to an external device (70, 71, 72, 73), **characterised in that** it comprises:
- a digital display (6);
- a circuit for wireless communication (82) with an external device;
- a manual selection interface (5, 6);
- a microphone (87);
- a processing unit (81) configured to:
∘ send a command to an external device (70, 71, 72, 73) based on an audio signal received by the microphone (87), via the wireless communication circuit;
∘ define a list of command options to be displayed, based on a list of previously selected commands, of environmental criteria, of behavioural criteria, of physiological criteria relating to the wearer of the timepiece, or of criteria relating to the state of the external device;
∘ display, on the digital display (6), a control interface comprising said command options (cmd1, cmd2, cmd3, cmd4, cmd5, cmd6, cmd7) from the defined list;
∘ identify the selection of a command displayed on the digital display, via the manual selection interface;
∘ send a command to an external device (70, 71, 72, 73) based on the identified selected command, via the wireless communication circuit (82),
**characterised in that** the processing unit (81) is configured to execute a machine learning algorithm capable of assisting in the dynamic generation, on the digital display (6), of said control interface comprising the command options, the processing unit (81) executing this algorithm can proactively generate and send at least one command to at least one external device, thereby avoiding any user interaction with the selection interface.

2. Timepiece (1) according to claim 1, **characterised in that** it comprises an interface for measuring the physiological data of a wearer of the timepiece (84), said processing unit (81) being configured to define the list of command options to be displayed based on the physiological criteria of the wearer of the timepiece.

3. Timepiece (1) according to claim 1 or 2, **characterised in that** it comprises a geolocation device (83), said processing unit (81) being configured to define the list of command options to be displayed based on the position of the timepiece as determined by the geolocation device (83).

4. Timepiece (1) according to any of the preceding claims, **characterised in that** it comprises a memory for saving one or more of the most recently sent commands, said processing unit (81) being configured to define the list of command options to be displayed by including one or more of the most recently saved commands or one or more commands that are counter to these most recent commands.

5. Timepiece (1) according to any of the preceding claims, **characterised in that** it comprises an accelerometer (85), said processing unit (81) being configured to define the list of command options to be displayed based on the accelerations measured by the accelerometer (85).

6. Timepiece (1) according to any of the preceding claims, **characterised in that** said processing unit (81) is configured to define the list of command options to be displayed based on a status criterion detected for an external device located in the vicinity of the timepiece.

7. Timepiece (1) according to any of the preceding claims, **characterised in that** the manual selection interface is a touch pad associated with the digital display (6).
